# EUROPEAN PATENT APPLICATION

(11) **EP 2 716 155 A2**
(43) Date of publication of application: **09.04.2014**
(21) Application number: 12788862.6
(22) Date of filing: 24.05.2012
(51) Int. Cl.: A01K 67/027, C12N 5/10, C12N 15/62, C12N 15/85, C12N 15/63

(54) **TRANSFORMED CAENORHABDITIS ELEGANS AND METHOD FOR SCREENING FOR SUBSTANCES REGULATING GLUCOSE METABOLISM USING SAME**

(30) Priority: 25.05.2011 KR 20110049519
(71) Applicant: Postech Academy-industry Foundation, Gyeongsangbuk-do 790-784 (KR)
(72) Inventor: LEE, Seung Jae, Phohang-si Gyeongsangbuk-do 790-751 (KR); LEE, Dong Yeop, Phohang-si Gyeongsangbuk-do 790-390 (KR)
(74) Representative: Price, Phillip
(86) International application number: PCT/KR2012/004133
(87) International publication number: WO 2012/161538

(57) **Abstract**

The present invention relates to a method for preparing a transformed *Caenorhabditis elegans* (*C. elegans*) which reacts to glucose by exhibiting fluorescence, and to a method for screening for a candidate substance and for a novel gene capable of regulating glucose metabolism and metabolic diseases using the transformed *Caenorhabditis elegans.* The transformed *Caenorhabditis elegans* of the present invention is prepared such that changes in glucose may be easily observed on a real-time basis using a fluorescent microscope. The transformed *Caenorhabditis elegans* of the present invention enables substances for regulating glucose metabolism to be quickly and accurately discovered, and further, may be expected to significantly contribute to studies on a variety of incurable metabolism-related diseases such as obesity, diabetes, and the like.

## Description

### Technical Field

The present invention relates to methods of preparing transformed *Caenorhabditis elegans* (hereinafter abbreviated as *C*. *elegans*) responding to sugar to exhibit fluorescence, and screening a candidate substance and a new gene, which can regulate sugar metabolism and a metabolic disease, using the same.

### Background Art

*C*. *elegans* can be grown *in vitro* in large numbers at once at a relatively low cost because of its easy gene manipulation and small size. *C*. *elegans* is suitable for experimental uses because it takes only approximately 3 days to become an adult through four stages, L1, L2, L3, and L4, after hatching from its egg. *C*. *elegans* has a simple structure, with only 959 cells excluding reproductive cells, and is transparent. For these reasons, it is easy to directly observe the inside of *C*. *elegans* through a microscope.

In addition, a cell lineage to an adult from an embryo has been completely identified. It is known from the genome project that the genome of *C*. *elegans* is three times that of yeast, and 1/3 to 1/5 that of a human. The genome of *C*. *elegans* is 40% similar to that of a human, and shares 75% of the 5,000 human disease genes that are known so far. Thus, it is widely considered as a good model system for the study of human diseases.

Recently, as the interest in metabolisms and metabolic diseases has intensively increased, metabolic studies using the genetics of *C*. *elegans* are rapidly progressing. Many genes discovered through these studies are also preserved in mammals. For example, homogenous genes for genes important to the metabolism of mammals such as genes for an insulin signaling pathway, genes for an mTOR signaling pathway, and genes for Tubby, SREBP, PPARγ, BAR (bile acid receptor), etc. are present in *C*. *elegans,* and their roles in overall metabolism have been actively studied.

Sugar is an essential source of energy for almost all living organisms on Earth including humans. However, foods having great amounts of sugars are closely related to obesity, heart diseases and, particularly, type II diabetes causing a disorder of glucose in the blood, and thus can cause serious medical problems.

In Korea, the mortality rate for diabetes in 2003 was 25 of every one hundred thousand people, which had increased by over 47% over the preceding 10 years, compared to 17 of every one hundred thousand people in 1994. Social costs of diabetes in the United States are enormous, estimated to be approximately 98 billion dollars in 1997. Thus, for the sake of reducing the socioeconomic cost of diabetes, studies on diabetes are very important. However, the exact cause of diabetes is difficult to find since diabetes is determined by a complex interaction of genetic and environmental factors. Thus, to identify a network of genes serving in diabetes and find a substance that can regulate sugar metabolism, the use of a simple animal model such as *C*. *elegans* can be effective. However, up to now, there have been no studies for screening a candidate gene and a candidate drug involved in sugar metabolism and diabetes using *C*. *elegans.*

### Disclosure

### Technical Problem

The present invention is directed to a method of preparing transformed *C*. *elegans* whose real-time change of sugar *in vivo* can be observed by fluorescence using the characteristics of *C*. *elegans,* such as a low cost and high efficiency, and screening a candidate gene and a candidate drug, which can regulate sugar metabolism using the same.

Specifically, the present invention established usefulness of the transformed *C. elegans* as a sugar reporter *in vivo* by proving that the transformed *C*. *elegans* is changed in fluorescence when genes involved in sugar and lipid metabolisms are manipulated.

### Technical Solution

According to an aspect of the present invention, there is provided transformed *C*. *elegans* that responds to sugar to exhibit fluorescence *in vivo.* The sugar includes glucose.

In one embodiment of the present invention, the transformed *C*. *elegans* has *far-3::GFP* DNA prepared by attaching a green fluorescence protein (GFP) to a gene whose expression is increased by sugar uptake, i.e., *far-3,* which is integrated on a chromosome thereof.

According to another aspect of the present invention, there is provided a method of preparing transformed *C*. *elegans* including integrating *far-3::GFP* DNA on a chromosome thereof by UV radiation.

According to still another aspect of the present invention, there is provided a method of screening a candidate gene and a candidate drug, which can regulate sugar metabolism, using transformed *C*. *elegans* that responds to sugar to exhibit fluorescence *in vivo.*

In one embodiment of the present invention, the screening method includes suppressing the expression of a gene for regulating sugar metabolism using RNA interference (RNAi) as soon as the transformed *C*. *elegans* uptakes sugar.

### Advantageous Effects

Transformed *C*. *elegans* is prepared to easily observe the change in glucose in real time by an actual fluorescence microscope, and thus a sugar metabolism regulatory substance can be found rapidly and exactly. In addition, highly effective studies are possible at a low cost due to the characteristics of *C*. *elegans.* Thus, the present invention is expected to considerably contribute to studies for treating various incurable diseases relating to metabolism such as obesity, diabetes, etc.

### Description of Drawings

FIG. 1 is a schematic diagram illustrating DNA integration in the preparation of transformed *C*. *elegans;*
FIG. 2A shows that *far-3::GFP* transformed *C*. *elegans* grown in a medium containing 2% glucose exhibits brighter fluorescence than the control, and FIG. 2B shows a quantified result of the fluorescence;
FIG. 3A shows that the fluorescence increased by glucose is further increased than the control when glycolysis is interrupted by *gpi-1* RNAi in the presence of glucose, and FIG. 3B shows a quantified result of the fluorescence;
FIG. 4A shows that the fluorescence increased by glucose is further increased than the control when synthesis of a fatty acid is interrupted by *pod-2* RNAi or *fasn-1* RNAi in the presence of glucose, and FIG. 4B shows a quantified result of the fluorescence; and
FIG. 5A shows that the fluorescence increased by glucose is decreased, compared to the control, when the expected expression of a glucose transporter, H17B01.1, is suppressed by RNAi in the presence of glucose, and FIG. 5B shows a quantified result of the fluorescence.

### Best Mode

The present invention provides transformed *C*. *elegans* which responds to sugar to exhibit fluorescence.

The term "*C*. *elegans"* used herein is the abbreviation of the scientific name *Caenorhabditis elegans,* which is a nematode that eats bacteria and lives in soil, and is widely used in genetic, molecular biological, and cell biological experiments.

The term "transformation" used herein means the change in genetic properties of an organism by DNA injected from an external environment.

In addition, the present invention provides a method of preparing transformed *C*. *elegans,* which includes integrating *far-3::GFP* DNA on a chromosome thereof by UV radiation.

The term "green fluorescence protein (GFP)" used herein is a protein expressed in cells to exhibit strong green fluorescence, and widely used in various fields for detecting the expression of a gene, particularly, due to ease of observation.

The term "transformed *C*. *elegans"* used herein is a sugar reporter fluorescent *C*. *elegans,* prepared by integration of *far-3::GFP* DNA, which is prepared by attaching a GFP to a gene whose expression is increased by sugar uptake, i.e*., far-3.*

In addition, the present invention provides a method of screening a candidate gene and a drug for regulating sugar metabolism using transformed *C*. *elegans* responding to sugar to exhibit fluorescence *in vivo.* The screening method uses RNAi.

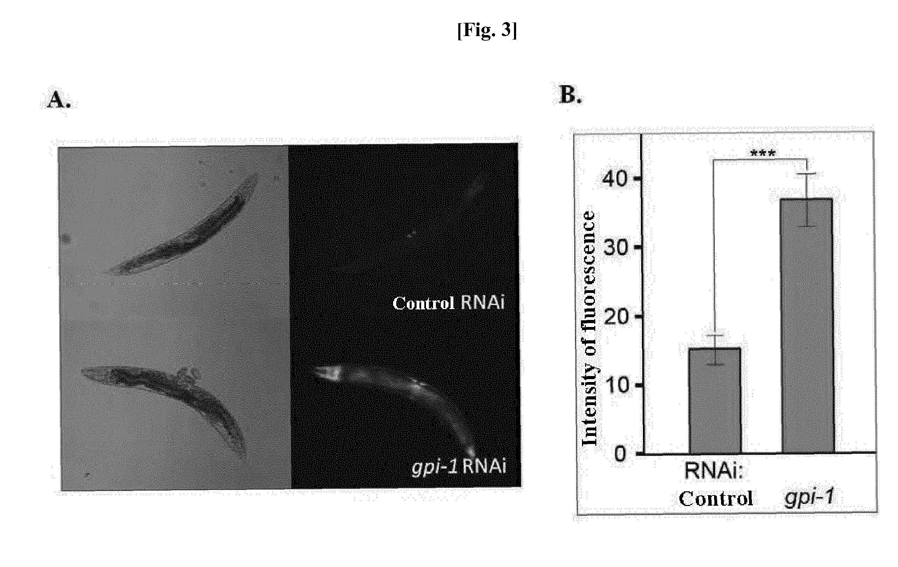

The method includes suppressing the expression of genes of *C*. *elegans* using RNAi as soon as the transformed *C*. *elegans* uptakes sugar. In one embodiment of the present invention, when lipid and sugar metabolic genes were suppressed, the significant change in intensity of green fluorescence was observed, which shows that a new gene involved in regulation of metabolism can be found using the transformed *C*. *elegans* (referred to FIGS. 3 to 5).

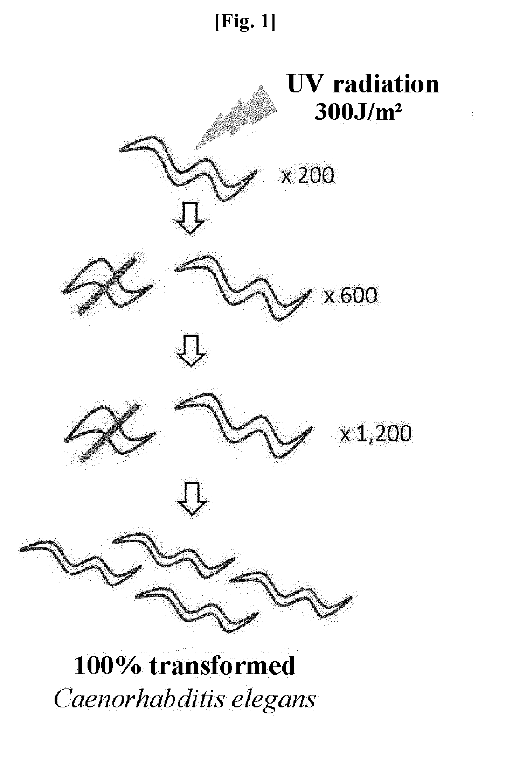

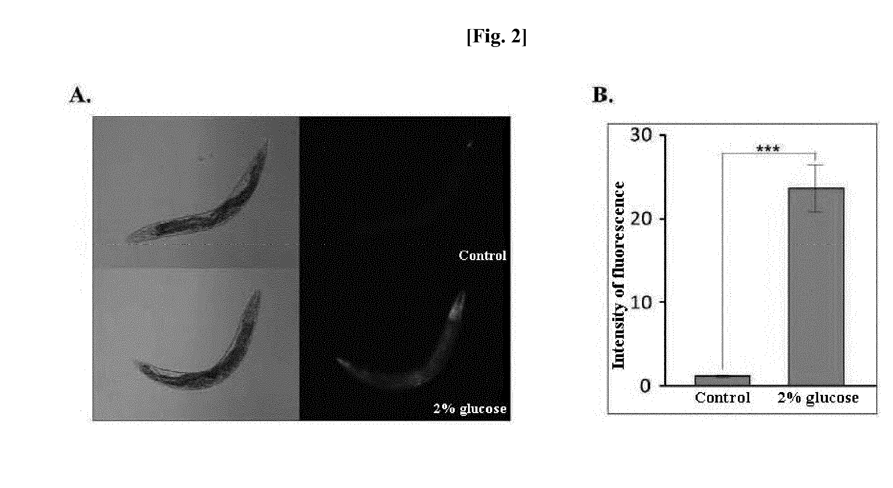

Specifically, the inventors prepared *far-3::GFP* transformed *C*. *elegans* prepared by attaching a GFP to a gene whose expression is increased by sugar uptake, i.e., *far-3,* to exhibit fluorescence, and then confirmed that, as shown in FIG. 1, *far-3::GFP* transformed *C*. *elegans* exhibited brighter fluorescence than the control when it was grown in a medium containing 2% glucose (refer to FIG. 2).

Subsequently, the inventors confirmed that the fluorescence increased by glucose was further increased than the control when glycolysis was interrupted by *gpi-1* RNAi in the presence of glucose (refer to FIG. 3).

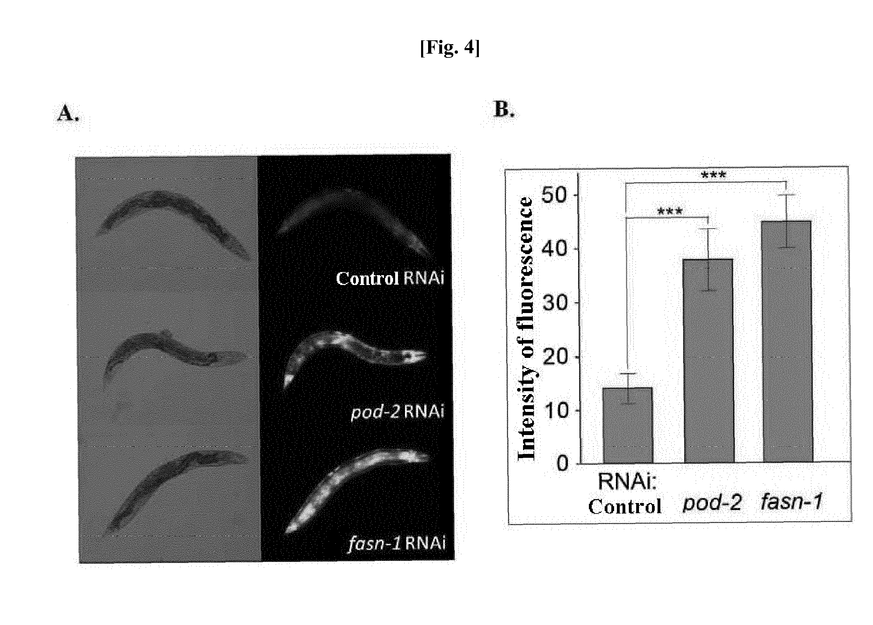

In addition, the inventors confirmed that the fluorescent increased by glucose was further increased than the control when the fatty acid synthesis was interrupted by *pod-2* RNAi or *fasn-1* RNAi in the presence of glucose (refer to FIG. 4).

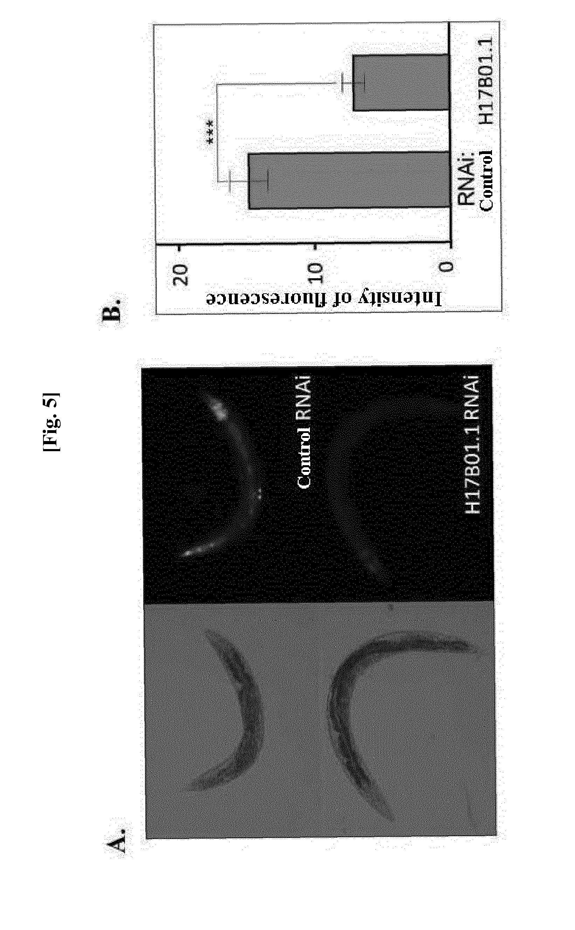

Moreover, the inventors confirmed that the fluorescence increased by glucose was reduced compared to the control when the expected expression of a sugar transporter, H17B01.1, was suppressed by RNAi in the presence of glucose (refer to FIG. 5).

Hereinafter, to help understanding of the present invention, preferable examples will be provided. However, the following examples are merely provided to more easily understand the present invention, not to limit the scope of the present invention.

### [Examples]

### Example 1. Method of preparing transformed C. elegans - DNA integration

Since *far-3::GFP* DNA inserted into *C*. *elegans* is a type that is not included in a chromosome, all of it is not delivered to offspring of *C*. *elegans.* For an experiment with such a type of transformed *C*. *elegans,* it is difficult to screen transformed *C*. *elegans,* and the expression of *far-3::GFP* is not uniform.

Accordingly, to obtain stable transformed *C*. *elegans,* the *far-3::GFP* DNA input form an external environment was integrated on a chromosome by changing DNA by UV radiation. The DNA integration by UV integration was performed at a very low probability, and thus a rate of success could be increased by UV radiation to a large number of *C*. *elegans.*

The *far-3::GFP* DNA was inserted with a gene recovering a short-body mutant (*dpy-5*) into a long body, and thus the transformation of *C*. *elegans* could be identified with an optical microscope. In the DNA integration, the transformed *C*. *elegans* was discriminated in the same manner as described above. As the result of measurement of a frequency of delivering the *far-3::GFP* DNA to an offspring before the DNA integration, it could be confirmed that the DNA was delivered to approximately 50% of the offspring, and it could be seen that the UV intensity showing approximately 50% viability after UV radiation was 300 J/m².

For a successful experiment for DNA integration, after the UV radiation, over 600 transformed *C*. *elegans* were needed, and thus 300 J/m² of UV radiation was applied to 200 transformed *C*. *elegans* in the L4 stage. After 6 days, 600 offspring of *C*. *elegans* (F1) born from the UV-treated *C*. *elegans* were transferred to 600 plates to produce offspring. After 4 days, two of each plate out of the offspring of *C. elegans* born from the 600 *C*. *elegans* (F2) were transferred into 1,200 plates to produce offspring. After approximately 4 days, the 1,200 plates were examined. Since the transformed *C*. *elegans* can be shown 100% if the DNA integration is successfully performed, a plate containing 100% normal-body *C*. *elegans* was searched for. 100% normal-body transformed *C*. *elegans* was confirmed by identifying the DNA integration by examining fluorescence from a medium containing glucose (referred to FIG. 1).

### Example 2. Measurement of fluorescence of transformed C. elegans of far-3::GFP in the presence of glucose

The *far-3::GFP* transformed *C*. *elegans* prepared in Example 1 was grown in a medium containing 2% glucose, and fluorescence was measured by the following method.

Specifically, a drop of 2% agarose was dropped on a slide glass, and covered with another side glass to spread the agarose thinly thereon. The slide glass was removed after the agarose was hardened to form a pad, and a drop of NaN₃ having a concentration of 50 mM was dropped on the agarose pad, and then approximately 15 *C*. *elegans* were transferred thereon to be anesthetized. A cover glass was carefully covered without air bubbles, and then fluorescence of *far-3::GFP* was observed by photographing an image using a fluorescence microscope. As the fluorescence microscope, Zeiss Axio Scope Al was used. The fluorescence of *C*. *elegans* exhibited in the entire body was quantified using an ImageJ program, and the fluorescence of over 20 *C*. *elegans* was analyzed by three independent experiments.

As shown in FIG. 2, it was confirmed that the fluorescence was brighter than that of the control, that is, *far-3::GFP* transformed *C*. *elegans,* grown in a medium without glucose (**P* < 0.05, ***P* < 0.01, ****P* <0.001, two-tailed Student's *t*-test).

### Example 3. Method of searching for sugar metabolism gene using RNAi

The inventors used a Julie Ahringer RNAi bacteria library to suppress the expression of a specific gene and to observe the fluorescence of *far-3::GFP.* Bacteria obtained from the library were streaked, transferred onto an LB/ampicillin plate, and incubated in an incubator at 37 °C for 12 to 16 hours. One bacteria colony grown on the plate was selected, transferred into a liquid LB/ampicillin medium, and incubated in a shaking culture incubator at 37 °C for 12 hours. After 12 hours, 100 µl of the LB/ampicillin medium in which bacteria were grown was dispensed into an NG medium including 2% glucose and ampicillin, and the bacteria were incubated in an incubator at 37 °C for 12 to 16 hours. For an induction reaction, 50 µl of isopropyl-1-thio-β-D-galactopyranoside (IPTG) having a concentration of 100 mM was dispensed onto the bacteria to react at room temperature for one day, and two adult *far-3::GFP* transformed *C*. *elegans* were transferred onto the plate in which RNAi bacteria were grown to lay eggs. After approximately 12 hours, the adult *far-3::GFP* transformed *C*. *elegans* was removed, and when *C*. *elegans* grown from the egg became an adult after three days, the expression of the fluorescence was observed using a method of measuring the fluorescence of the *far-3::GFP* by a fluorescence microscope.

### Example 3-1. Use of gpi-l RNAi

The inventors observed the change of intensity of fluorescence in *C*. *elegans* transformed when the expression of *gpi-I* genes known to be related to glycolysis was interrupted using *gpi-l* RNAi.

When glucose is input into a cell, it is converted into glucose-6-phosphate by a hexokinase, and then is converted into fructose-6-phosphate by a glucose-6-phosphate isomerase (*gpi-l*)*.* The *gpi-l* serves an important role in a high step of glycolysis and thus can interrupt the glycolysis through the suppression of the expression of *gpi-l.*

As shown in FIG. 3, it was seen that, when the expression of a *gpi-1* gene was interrupted, the fluorescence increased by glucose was further increased than in the control, that is, transformed *C*. *elegans,* in which the *gpi-1* expression was not decreased, in the presence of glucose (**P* < 0.05, ***P* < 0.01, ****P* <0.001, two-tailed Student's *t*-test).

### Example 3-2. Use of pod-2 RNAi or fasn-1 RNAi

The inventors observed the change of the intensity of the fluorescence of the transformed *C*. *elegans* when the expression of an acetyl-CoA carboxylase (*pod-2*) or a fatty acid synthase (*fasn-1*) known to be related to synthesis of fatty acids was interrupted using *pod-2* RNAi or *fasn-1* RNAi.

The *pod-2* is an enzyme located in the highest stage of the synthesis of fatty acids, serving to convert acetyl-CoA into malonyl-CoA. The *fasn-1* is involved in the synthesis of fatty acids using acetyl-CoA and malonyl-CoA in the next stage of *pod-2.* The *pod-2* and *fasn-1* are major enzymes to regulate the initial stage of the synthesis of fatty acids, which can be considerably suppressed by interrupting *pod-2* and *fasn-1.*

As shown in FIG. 4, it can be seen that the fluorescence increased by glucose was further included when the expression of a *pod-2* or *fasn-1* gene was interrupted, compared to the control, that is, transformed *C*. *elegans* in which the expression of a *pod-2* or *fasn-1* gene was not increased, in the presence of glucose (**P* < 0.05, ***P* < 0.01, ****P* <0.001, two-tailed Student's *t*-test).

### Example 3-3. Use of H17B01.1 RANi

The inventors observed the change of fluorescence intensity in transformed *C*. *elegans* when the expected expression of a glucose transporter, H17B01.1, was interrupted using H17B01.1 RANi.

H17B01.1 is expected to be a gene of *C*. *elegans* having the most similar base sequence to a human glucose transporter, GLUI1, serving to transport glucose into cells from *C*. *elegans.*

As shown in FIG. 5, it was seen that the fluorescence increased by glucose was decreased when the expression of H17B01.1 was interrupted, compared to the control, that is, transformed *C*. *elegans* in which the expression of H17B01.1 was interrupted, in the presence of glucose (**P* < 0.05, ***P* < 0.01, ****P* <0.001, two-tailed Student's *t*-test).

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

### Industrial Applicability

Transformed *C*. *elegans* enable a substance for regulating sugar metabolism to be found rapidly and exactly, and furthermore it is expected to considerably contribute to studies for treating various incurable diseases relating to metabolism such as obesity, diabetes, etc.

## Claims

1. A transformed *Caenorhabditis elegans* (hereinafter, abbreviated as *"C. elegans"),* which responds to sugar to exhibit fluorescence *in vivo.*

2. The transformed *C*. *elegans* according to claim 1, further comprising *far-3::GFP* DNA in which a green fluorescence protein (GFP) is attached to a gene whose expression is increased by sugar uptake, *far-3,* integrated on a chromosome thereof.

3. A method of preparing transformed *C*. *elegans,* comprising:
integrating *far-3::GFP* DNA on a chromosome thereof by UV radiation.

4. A method of screening a candidate gene and drug for regulating sugar metabolism, which uses transformed *C*. *elegans* responding to sugar to exhibit fluorescence *in vivo.*

5. The method according to claim 4, which comprises:
suppressing the expression of the gene for regulating sugar metabolism using RNA interference (RNAi) as soon as the transformed *C*. *elegans* uptakes sugar.
